# EUROPEAN PATENT APPLICATION

(11) **EP 4 265 209 A1**
(43) Date of publication of application: **25.10.2023**
(21) Application number: 23168182.6
(22) Date of filing: 17.04.2023
(51) Int. Cl.: A61B 18/12, A61B 18/00, A61B 18/14

(54) **A SWITCHING UNIT FOR OPERATING A MULTI-CATHETER SYSTEM**

(30) Priority: 18.04.2022 US 202217722664
(71) Applicant: BIOSENSE WEBSTER (ISRAEL) LTD., 2066717 Yokneam (IL)
(72) Inventor: GOVARI, Assaf, 2066717 Yokneam (IL); GLINER, Vadim, 2066717 Yokneam (IL); ALTMANN, Andres Claudio, 2066717 Yokneam (IL)
(74) Representative: Carpmaels & Ransford LLP

(57) **Abstract**

A system includes: (a) a switching unit including: (i) first sockets, which are configured to connect between the switching unit and channels of one or more catheters, (ii) second sockets, which are configured to connect, via the switching unit, between the first sockets and a console, and (iii) a switch, which is configured, based on a control signal, to route signals conducted in the channels, between the first and second sockets, and (b) circuitry, which is configured, based on information received from the one or more catheters, to produce the control signal for routing of the signals.

## Description

### FIELD OF THE DISCLOSURE

The present disclosure relates generally to medical devices, and particularly to methods and systems for operating a medical system having multiple catheters.

### BACKGROUND OF THE DISCLOSURE

Various techniques for operating medical catheterization systems have been published.

For example, U.S. Patent Application Publication 2018/0168729 describes a catheter system for ablating a tissue portion of a body and visualizing the ablation in real time.

The present disclosure will be more fully understood from the following detailed description of the examples thereof, taken together with the drawings in which:

### BRIEF DESCRIPTION OF THE DRAWINGS

Fig. 1 is a schematic, pictorial illustration of a catheter-based tracking and ablation system, in accordance with an example of the present disclosure;
Fig. 2 is a schematic, pictorial illustration of a switching unit (SU) of the system of Fig. 1, in accordance with an example of the present disclosure; and
Fig. 3 is a flow charts that schematically illustrate a method for operating a multi-catheter system using the SU of Fig. 2, in accordance with examples of the present disclosure.

### DETAILED DESCRIPTION OF EXAMPLES

### OVERVIEW

Some medical procedures, such as electrophysiology (EP), require using multiple catheters of an EP system, each of which typically has multiple channels. An operating console of the EP system typically has a fixed number of sockets and channels available for the catheters. In some cases, there is a mismatch between the number of sockets and/or channels (i) required for the catheters and (ii) available in the console. In such cases, the user of the EP system may have to unplug a given catheters from the console, and subsequently, to re-plug the given catheter to the console, which is time consuming and interferes with the EP procedure.

Examples of the present disclosure that are described hereafter provide improved techniques for operating a medical system having multiple catheters and managing all the channels without forcing the user to handle the unplugging and re-plugging of catheters during the procedure.

In some examples, a system having a plurality of multi-channel catheters comprises a switching unit, and circuitry. The switching unit comprises (i) first sockets, which are configured to connect between the switching unit and the channels of one or more catheters, and (ii) second sockets, which are configured to connect, via the switching unit, between the first sockets a console.

In some examples, the system comprises memory devices, such as electrically erasable programmable read-only memory (EEPROM) devices, each of which is coupled to a respective catheter of the multi-channel catheters. The EEPROM is configured to store information about the channels of the respective catheter. The information may comprise, for example, properties of the signal, and the source and destination of the signal of each channel.

In some examples, the circuitry is configured, based on information received from the EEPROM devices of the one or more catheters, to produce a control signal for routing the signals. In other words, the circuitry is configured to provide instructions to the switching unit on how to route the signals for each channel of each catheter plugged into the first sockets.

In some examples, the switching unit comprises a switch, which is configured, based on the control signal received from the circuitry to route signals conducted in the channels between the first and second sockets, so that each signal is routed between the source and destination stored in the respective EEPROM device.

In some examples, the console comprises third sockets, so that the first, second, and third sockets comprise first, second and third interconnects, respectively, which are configured to conduct the signals. In some examples, the number of the first interconnects may differ from the number of the second interconnects, and the number of the third interconnects is similar to the number of the second interconnects. In such examples, the user of the system can plug any number of catheters into the first sockets of the switching unit, and the signals are automatically routed between the source and the destination.

In some examples, when first and second catheters are plugged into the switching unit, the circuitry is configured to detect that the first catheter is active and the second catheter is inactive, and responsively, to produce the control signal that controls the switching unit to connect between the first catheter and the console, and to disconnect between the second catheter and the console. Moreover, the circuitry is configured to control the switching unit to carry out the connecting and disconnecting in a channel level. In other words, to connect active channels, and to temporary disconnect inactive channels. Note that whenever a given channel and/or a given catheter is active (e.g., intended to send and/or receive a signal), the circuitry is configured to re-connect the given channel and/or catheter.

The disclosed techniques improve the versatility and ease-of-use of multi-catheter medical systems, and improve the robustness of signal transmission during medical procedures that require using a plurality of multi-channel catheters.

### SYSTEM DESCRIPTION

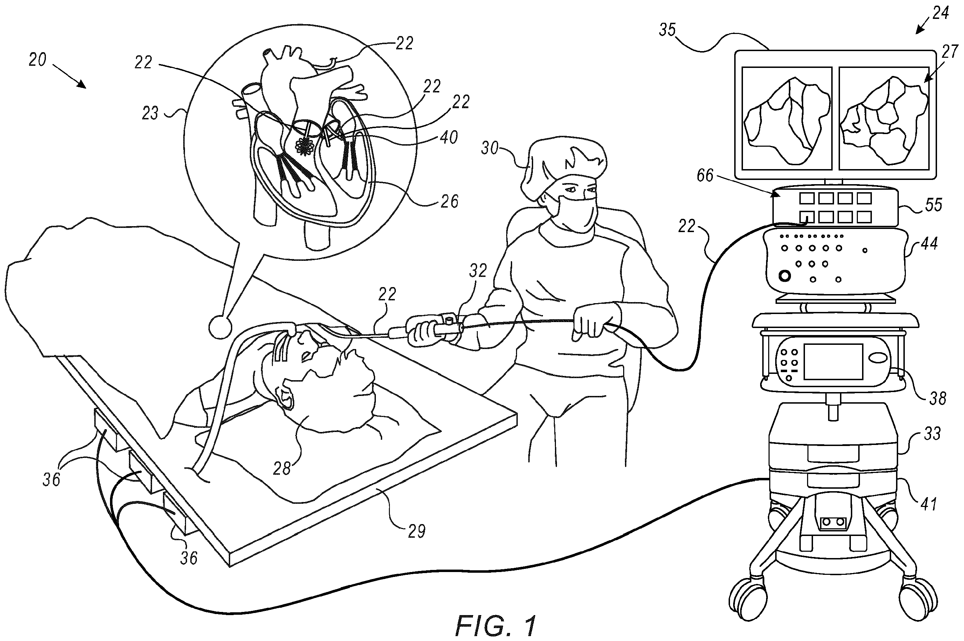

Fig. 1 is a schematic, pictorial illustration of a catheter-based tracking and ablation system 20, in accordance with an example of the present disclosure.

In some examples, system 20 comprises a multi-catheter system configured to operate a plurality of one or more types of catheters. In the present example, system 20 comprises multiple cardiac catheters 22, which are configured to carry out cardiac procedures, and a control console 24. In the example described herein, catheters 22 may be used for any suitable therapeutic and/or diagnostic purposes, such as sensing electro-anatomical signals and/or ablation of tissue in a heart 26.

In some examples, console 24 comprises a processor 33, typically a general-purpose computer, with suitable front end and interface circuits for receiving signals via catheters 22 and for controlling the other components of system 20 described herein. Console 24 further comprises a user display 35, which is configured to receive from processor 33 graphical and/or textual display items, such as a map 27 of heart 26, and to display map 27.

In some examples, map 27 may comprise any suitable type of three-dimensional (3D) anatomical map produced using any suitable technique. For example, the anatomical map may be produced using an anatomical image produced by using a suitable medical imaging system, or using a fast anatomical mapping (FAM) technique available in the CARTO^{™} system, produced by Biosense Webster Inc. (Irvine, Calif.), or using any other suitable technique, or using any suitable combination of the above.

In some examples, console 24 comprises a recording unit 38, which is configured to record in case of failure in the CARTO^{™}and/or a failure to pace in certain electrodes. a patient interface unit (PIU) 44, which is configured to produce a signal indicative of the location and electrocardiogram (ECG) signals that are acquired and processed.

In some examples, system 20 comprises a switching unit (SU) 55, which is connected to PIU 44 of console 24, and has one or more sockets 66 for connecting with catheters 22, respectively. In the example of Fig. 1, only one catheter 22 is connected to a respective socket 66, for the sake of clarity. Typically, the proximal end of all catheters 22 are connected to respective sockets 66 of SU 55.

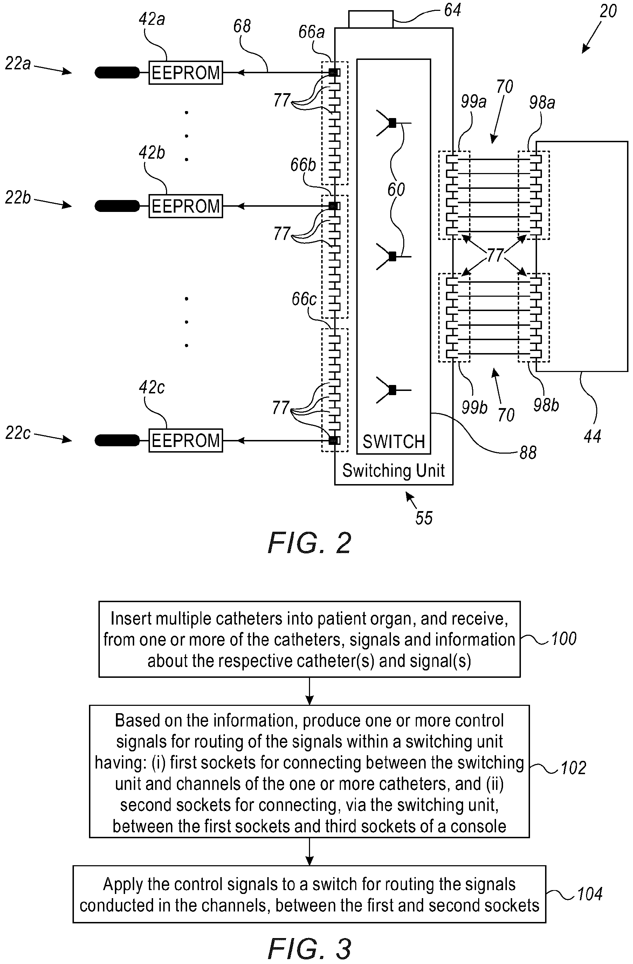

In some examples, SU 55 is configured to route signals between catheters 22 and console 24. The structure and functionality of SU 55 are described in detail in Fig. 2 below.

In other examples, the PIU and the switching unit may be integrated together, so that the user of system 20 is plugging (and unplugging) the catheters and other components to the integrated entity.

Reference is now made to an inset 23. In some examples, prior to performing an ablation procedure, a physician 30 inserts one or more catheters, such as catheter 22, through the vasculature system of a patient 28 lying on a table 29, so as to perform electro-anatomical (EA) mapping of tissue in question of heart 26.

In some examples, catheter 22 comprises a distal-end assembly 40 having multiple sensing electrodes (not shown). For example, distal-end assembly 40 may comprise: (i) a basket catheter having multiple splines, each spline having multiple sensing electrodes, (ii) a balloon catheter having multiple sensing electrodes disposed on the surface of the balloon, or (iii) a focal catheter having multiple sensing electrodes. Each sensing electrode is configured to produce, in response to sensing electrophysiological (EP) signals in tissue of heart 26, one or more signals indicative of the sensed EP signals.

In the example of Fig. 1, three catheters 22 are inserted into heart 26, but in other examples, any other suitable number of catheters may be inserted into an organ in question. For example, the catheterization procedure may require the insertion of between about 5 and 8 catheters. Moreover, different type of catheters may be inserted and used at the same time, for example: a balloon or basket catheter (for performing sensing and/or ablation at multiple locations), together with a focal catheter (for sensing or ablating tissue at a single position).

In some examples, the proximal end of catheter 22 is connected, *inter alia,* to interface circuits (not shown) of PIU, or to SU 55 (as shown in the example of Fig. 1), so as to transfer these signals to processor 33 for performing the EA mapping.

In the context of the present disclosure and in the claims, the terms "about" or "approximately" for any numerical values or ranges indicate a suitable dimensional tolerance that allows the part or collection of components to function for its intended purpose as described herein.

In other examples, catheter 22 may comprise one or more ablation electrodes (not shown) coupled to distal-end assembly 40. The ablation electrodes are configured to ablate tissue at a target location of heart 26, which is determined based on the analysis of the EA mapping of the tissue in question of heart 26. After determining the ablation plan, physician 30 navigates distal-end assembly 40 in close proximity to the target location in heart 26 e.g., using a manipulator 32 for manipulating catheter 22. Subsequently, physician 30 places one or more of the ablation electrodes (of a selected catheter) in contact with the target tissue, and applies, to the tissue, one or more ablation signals. Additionally, or alternatively, physician 30 may use any different sorts of suitable catheters for ablating tissue of heart 26 so as to carry out the aforementioned ablation plan.

In some examples, the position of distal-end assembly 40 in the heart cavity is measured using a position sensor (not shown) of a magnetic position tracking system. In the present example, console 24 comprises a driver circuit 41, which is configured to drive magnetic field generators 36 placed at known positions external to patient 28 lying on table 29, e.g., below the patient's torso. The position sensor is coupled to the distal end, and is configured to generate position signals in response to sensed external magnetic fields from field generators 36. The position signals are indicative of the position the distal end of catheter 22 in the coordinate system of the position tracking system.

This method of position sensing is implemented in various medical applications, for example, in the CARTO^{™} system, produced by Biosense Webster Inc. (Irvine, Calif.) and is described in detail in U.S. Patents 5,391,199, 6, 690, 963, 6,484,118, 6, 239, 724, 6, 618, 612 and 6, 332, 089, in PCT Patent Publication WO 96/05768, and in U.S. Patent Application Publications 2002/0065455 A1, 2003/0120150 A1 and 2004/0068178 A1, whose disclosures are all incorporated herein by reference.

In some examples, the coordinate system of the position tracking system is registered with the coordinate systems of system 20 and map 27, so that processor 33 is configured to display, the position of distal-end assembly 40, over the anatomical or EA map (e.g., map 27).

In some examples, processor 33, typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This particular configuration of system 20 is shown by way of example, in order to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other sorts of medical systems.

### SWITCHING UNIT FOR ROUTING SIGNALS FROM MULTIPLE CATHETERS

Fig. 2 is a schematic, pictorial illustration of switching unit (SU) 55 connecting between catheters 22 and PIU 44 of console 24, in accordance with an example of the present disclosure.

In some examples, SU 55 comprises sockets 66a, 66b and 66c, which are configured to connect between SU 55 and channels of one or more catheters. In the present example, three catheters, 22a, 22b and 22c are connected to SU 55 via sockets 66a, 66b and 66c, respectively. In the present example, each socket 66 comprises about eight (8) channels 77, but in other examples, each socket 66 may have any suitable number of channels (e.g., socket 66a may have about eight channels 77, and socket 66b may have about twenty (20) channels 77). Note that catheters 22a, 22b and 22c comprise suitable plug, which are configured to snugly fit into sockets 66a, 66b and 66c, respectively. Each socket and plug may have any suitable number of channels configured to exchange signals between catheters 22a, 22b and 22c and sockets 66a, 66b and 66c, respectively.

In some examples, SU 55 comprises sockets 99, which are configured to connect, via SU 55, between sockets 66 and PIU 44 of console 24. In other examples, PIU 44 may be integrated with SU 55, so that sockets 99 are configured to connect between sockets 66 and console 24 directly. In the present example, SU 55 comprises a socket 99a having about four (4) channels 77 and a socket 99b having about six (6) channels 77. Moreover, in the example of Fig. 2, PIU 44 of console 24 (or any other interface of console 24) has sockets 98a and 98b.

In some examples, sockets 99a and 98a have the same number of channels 77 (e.g., about 4 channels), and sockets 99b and 98b have the same number of channels 77 (e.g., about 6 channels). In the present example, the connection between SU 55 and PIU 44 comprises one-to-one connection, which is enabled by the same number of channels. In such examples, each pair of channels is interconnected using a cable 70.

In other examples, instead of a pair of sockets (e.g., sockets 99a and 98a) and cable 70, system 20 comprises a pair of plug and socket. For example, a plug instead of socket 99a, which is configured to fit into socket 98a, or *vice versa,* so as to conduct signals in channels 77, between SU 55 and PIU 44.

In yet other examples, instead of one or both of sockets 66 and 99, SU 55 may comprise respective plugs. For example, at least one of catheters 22 (and/or cables 68) may have a socket and SU 55 may have a suitable plug for connecting between the respective catheter 22 and SU 55.

In some examples, each catheter 22 may have between about 3 and 500 channels 77. For example, a focal catheter 22 may have about 3 channels 77 (e.g., for a position sensor, and one or two electrocardiogram (ECG) and/or temperature sensors), whereas a multi-electrode basket catheter 22 may have about 500 channels 77 (e.g., for position sensors, temperature sensors, ECG sensors, contact force sensors, and for ablation electrodes).

In some examples, at least one of, and typically each catheter 22, comprises a memory device. In the present example, catheters 22a, 22b and 22c comprise electrically erasable programmable read-only memory (EEPROM) devices 42a, 42b and 42c, respectively. EEPROM device 42 of respective catheter 22 is configured to store information related to the respective catheter 22, such as but not limited to catheter identification, type of signals intended to be conducted in each channel of the catheter, and the destination of each signal).

In some examples, system 20 comprises circuitry 64, which is configured to receive from each EEPROM device 42, information about the number of channels 77, the type of signal, and the destination of each signal conducted in each respective channel 77. In the present example, the information is received over cables 68 of catheters 22, and for each catheter 22 and respective socket 66, one of channels 77 may be allocated to the information received from the respective EEPROM device 42.

In some examples, circuitry 64 is configured, to receive the information from EEPROM devices 42a, 42b and 42c, of catheters 22a, 22b, and 22c, respectively. Based on information received from EEPROM devices 42, circuitry 64 is configured to produce a control signal for routing the signals between sockets 66 and 99, so as to route the signals between catheters 22 and console 24. In other words, circuitry 64 is configured to provide instructions to SU 55 on how to route the signals for each channel of each catheter plugged into sockets 66.

In some examples, SU 55 comprises a switch 88, which is configured, based on the control signal, to route the signals conducted in channels 77, between sockets 66 and 99. In the present example, switch 88 comprises about three internal switches (ISs) 60, configured to perform the signal routing between each channel 77 and a respective channel 99.

In some cases, at least one of catheters 22 (e.g., catheter 22a) is active and at least one of catheters 22 (e.g., catheter 22c) is inactive. In some examples, based on the information received from EEPROM devices 42, circuitry 64 is configured to detect that catheter 22a is active and catheter 22c is inactive. In such examples, circuitry 64 is configured to produce the control signal that controls switch 88 of SU 55 to connect between the catheter 22a and console 24, and to disconnect between catheter 22c and console 24. Note that in response to receiving from EEPROM device 42c a signal indicative of catheter 22c being activated, circuitry 64 is configured to produce an additional control signal that controls switch 88 of SU 55 to reconnect between the catheter 22c and console 24.

In some examples, circuitry 64, typically comprises a general-purpose processing device, which is programmed in software to carry out the functions described herein. The software may be downloaded to the processing device in electronic form, over a network, for example, or it may, alternatively or additionally, be provided and/or stored on non-transitory tangible media, such as magnetic, optical, or electronic memory.

Fig. 3 is a flow charts that schematically illustrate a method for operating system 20 using SU 55, in accordance with examples of the present disclosure.

The method begins at a catheter insertion step 100, with physician 30 inserting multiple catheters 22 into patient organ (e.g., heart 26), and circuitry 64 receiving from EEPROM devices 42, signals and information about the respective catheters 22 and signals, as described in detail in Fig. 2 above.

At a control signal generation step 102, based on the information received from EEPROM devices 42, circuitry 64 produces one or more control signals for routing of the signals within SU 55 having: (i) sockets 66 for connecting between SU 55 and channels of catheters 22, and (ii) sockets 99 for connecting, via SU 55, between the sockets 66 and sockets 98 of PIU 44 of console 24. The control signal generation and the structure and functionality of SU 55 are described in detail in Fig. 2 above.

At a signal routing step 102 that concludes the method, the control signals are applied to switch 88 for routing the signals conducted in channels 77, between the sockets 66 and 99. In some examples, the control signals comprise routing instructions for each of the signals received from each channel of catheters 22. Based on the control signals, switch 88 is configured to route the respective signals (via the channels defined in the control signals) to the destination within console 24. For example, signals from a position sensor are routed to a position tracking system or module, ECG signals may be routed to processor 33, and ablation signals may be routed from a radiofrequency (RF) generator (not shown) that is implemented, e.g., in console 24, to ablation electrodes of a respective catheter 22.

In some examples, physician 30 plugs all catheters 22 into sockets 66 of SU 55 and SU 55 routes the channels from the different catheters 22 to PIU 44 or to other parts of system 20 as needed (e.g., an RF generator, or an irreversible electroporation (IRE) generator, or processor 33). Note that the configuration of the hardwire connection between SU 55 and PIU 44 (e.g., sockets 99 and 98), as well as any other parts of system 20 (e.g., the IRE generator and processor 33) do not change and are not affected by the type of catheters 22 and number of channels 77 of each catheter 22 plugged into sockets 66.

In some examples, in steps 102 and 104, in response to detecting that catheter 22a is active and catheter 22c is inactive, circuitry 64 is configured to produce the control signal that controls switch 88 of SU 55 to connect between catheter 22a and console 24, and to disconnect between catheter 22c and console 24, as described in Fig. 2 above.

Although the examples described herein mainly address electrophysiology procedures, the methods and systems described herein can also be used in other applications, such as in any catheterization procedure using multiple catheters or probes having multiple signals intended to be routed between the catheters and other parts of the respective medical system.

### Example 1

A system (20) including:
(a) A switching unit (55) including: (i) first sockets (66a, 66b, 66c), which are configured to connect between the switching unit (55) and channels (77) of one or more catheters (22a, 22b, 22c), (ii) second sockets (99a, 99b), which are configured to connect, via the switching unit (55), between the first sockets (66a, 66b, 66c) and a console (24), and (iii) a switch (88), which is configured, based on a control signal, to route signals conducted in the channels (77), between the first sockets (66a, 66b, 66c) and second sockets (99a, 99b), and
(b) Circuitry (64), which is configured, based on information received from the one or more catheters (22a, 22b, 22c), to produce the control signal for routing of the signals.

### Example 2:

The system according to Example 1, wherein the console includes third sockets, wherein the first, second, and third sockets include first, second and third interconnects, respectively, which are configured to conduct the signals, and wherein the first sockets include a first number of the first interconnects, different from a second number of the second interconnects.

### Example 3:

The system according to Example 2, wherein the third sockets have the second number of the third interconnects.

### Example 4:

The system according to Example 1, wherein at least one of the catheters includes a memory device, which is configured to store the information.

### Example 5:

The system according to Example 4, wherein the memory device includes an electrically erasable programmable read-only memory (EEPROM).

### Example 6:

The system according to Example 1, wherein the catheters include first and second catheters, wherein, based on the information, the circuitry is configured to: (i) detect that the first catheter is active and the second catheter is inactive, and (ii) produce the control signal that controls the switching unit to connect between the first catheter and the console, and to disconnect between the second catheter and the console.

### Example 7

A method, including:
receiving, from one or more catheters, signals and information, and based on the information, producing a control signal for routing of the signals within a switching unit having: (i) first sockets for connecting between the switching unit and channels of the one or more catheters, and (ii) second sockets for connecting, via the switching unit, between the first sockets a console; and
applying the control signals to a switch for routing the signals conducted in the channels, between the first and second sockets.

### Example 8:

The method according to Example 7, wherein the console includes third sockets, wherein the first, second, and third sockets include first, second and third interconnects, respectively, for conducting the signals, and wherein the first sockets include a first number of the first interconnects, different from a second number of the second interconnects.

### Example 9:

The method according to Example 8, wherein the third sockets have the second number of the third interconnects

### Example 10:

The method according to Example 7, wherein at least one of the catheters includes a memory device for storing the information, and wherein the information is received from the memory device.

### Example 11:

The method according to Example 10, wherein the memory device includes an electrically erasable programmable read-only memory (EEPROM).

### Example 12:

The method according to claim 7, wherein the catheters include first and second catheters, wherein, receiving the information includes detecting that the first catheter is active and the second catheter is inactive, and producing the control signal includes controlling the switching unit to connect between the first catheter and the console, and to disconnect between the second catheter and the console.

It will thus be appreciated that the examples described above are cited by way of example, and that the present disclosure is not limited to what has been particularly shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A system, comprising:
a switching unit, comprising:
first sockets, which are configured to connect between the switching unit and channels of one or more catheters;
second sockets, which are configured to connect, via the switching unit, between the first sockets and a console; and
a switch, which is configured, based on a control signal, to route signals conducted in the channels, between the first and second sockets; and
circuitry, which is configured, based on information received from the one or more catheters, to produce the control signal for routing of the signals.

2. The system according to claim 1, wherein the console comprises third sockets, wherein the first, second, and third sockets comprise first, second and third interconnects, respectively, which are configured to conduct the signals, and wherein the first sockets comprise a first number of the first interconnects, different from a second number of the second interconnects.

3. The system according to claim 2, wherein the third sockets have the second number of the third interconnects.

4. The system according to claim 1, wherein at least one of the catheters comprises a memory device, which is configured to store the information.

5. The system according to claim 4, wherein the memory device comprises an electrically erasable programmable read-only memory (EEPROM).

6. The system according to claim 1, wherein the catheters comprise first and second catheters, wherein, based on the information, the circuitry is configured to: (i) detect that the first catheter is active and the second catheter is inactive, and (ii) produce the control signal that controls the switching unit to connect between the first catheter and the console, and to disconnect between the second catheter and the console.

7. A method, comprising:
receiving, from one or more catheters, signals and information, and based on the information, producing a control signal for routing of the signals within a switching unit having: (i) first sockets for connecting between the switching unit and channels of the one or more catheters, and (ii) second sockets for connecting, via the switching unit, between the first sockets and a console; and
applying the control signals to a switch for routing the signals conducted in the channels, between the first and second sockets.

8. The method according to claim 7, wherein the console comprises third sockets, wherein the first, second, and third sockets comprise first, second and third interconnects, respectively, for conducting the signals, and wherein the first sockets comprise a first number of the first interconnects, different from a second number of the second interconnects.

9. The method according to claim 8, wherein the third sockets have the second number of the third interconnects.

10. The method according to claim 7, wherein at least one of the catheters comprises a memory device for storing the information, and wherein the information is received from the memory device.

11. The method according to claim 10, wherein the memory device comprises an electrically erasable programmable read-only memory (EEPROM).

12. The method according to claim 7, wherein the catheters comprise first and second catheters, wherein, receiving the information comprises detecting that the first catheter is active and the second catheter is inactive, and producing the control signal comprises controlling the switching unit to connect between the first catheter and the console, and to disconnect between the second catheter and the console.
